Europäisches Patentamt

European Patent Office

Office européen des brevets

(19)

(11) Veröffentlichungsnummer: **0 139 981**
**B1**

(12) # EUROPÄISCHE PATENTSCHRIFT

(45) Veröffentlichungstag der Patentschrift:
06.08.86

(51) Int. Cl.⁴: **C 07 C 29/80,** C 07 C 31/125

(21) Anmeldenummer: 84109962.5

(22) Anmeldetag: 22.08.84

(54) Verfahren zur destillativen Aufarbeitung von Wasser und Methanol enthaltenden höheren Alkoholen mit 6 bis 20 C-Atomen.

(30) Priorität: 22.10.83 DE 3338439

(43) Veröffentlichungstag der Anmeldung:
08.05.85 Patentblatt 85/19

(45) Bekanntmachung des Hinweises auf die Patenterteilung:
06.08.86 Patentblatt 86/32

(84) Benannte Vertragsstaaten:
AT BE CH DE FR GB IT LI NL

(56) Entgegenhaltungen:
US - A - 2 658 083

(73) Patentinhaber: **HÜLS AKTIENGESELLSCHAFT,
Patentabteilung / PB 15 - Postfach 13 20,
D-4370 Marl 1 (DE)**

(72) Erfinder: **Sridhar, Srinivasan, Dr., Lehmbecker Pfad 52,
D-4370 Marl (DE)**
Erfinder: **Hartmann, Manfred, Hülsmannsfeld 40,
D-4370 Marl (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Beschreibung

Die Herstellung von Fettalkoholen aus Fettsäuren kann auf zwei verschiedene Weisen erfolgen: Durch Veresterung der Säuren zu Methylester und anschliessende Hydrierung zu den Alkoholen bzw. durch Direkthydrierung (Chem. Eng. 21.02.83, S. 42). Das entstandene Prozesswasser wird, sofern es als Wasserphase vorliegt, abdekantiert. Der in den Alkoholen gelöst vorliegende Anteil an Wasser wird destillativ abgetrennt. Das Destillatwasser wird von leicht siedenden Stoffen wir Kohlenwasserstoffe, Ester und dergleichen sowie von nicht abreagiertem Methanol begleitet. Auch die kontinuierliche Abtrennung von entstehendem Wasser und von überschüssigem Methanol bei simultaner Veresterung in einer Reaktionskolonne erfolgt ebenfalls gemeinsam. Das Prozesswasser kann ferner auch azeotrop mit Butanol (FR-PS 1 493 754 = GB-PS 1 173 217) abdestilliert werden.

Bei einem weiteren Verfahren zur Aufarbeitung von aus Synthesegas hergestellten $C_1$- bis $C_8$-Alkoholen (SU-PS 480 693) sowie Kohlenwasserstoffen wird als erster Schritt die wasserhaltige homogene Phase, nach der Hydrierung, durch gemeinsame Verdampfung von $C_1$- bis $C_5$-Alkoholen und Wasser befreit.

Ein weiteres Verfahren beschreibt die destillative Abtrennung von Wasser und Kohlenwasserstoffen in drei Kolonnen (CA 88 (1978) 52282 e).

Alle diese Verfahren haben den gemeinsamen Nachteil, dass das Wasser kostenaufwendig destillativ abgetrennt wird.

Da die Fettalkohole bei Temperaturen oberhalb 250°C thermisch labil sind, muss die Destillation bei einem Kopfdruck von ca. 400 mbar erfolgen. Dies erfordert eine Vakuumanlage und den Einsatz von Kälte. Ferner wird bei grösseren Anteilen an gelöstem Wasser ($\geq 5$ Gew.-%) die Destillation durch Siedeverzüge erheblich erschwert. Das destillativ abgetrennte Wasser enthält — auch nach weiterer destillativen Abtrennung von Methanol — flüchtige organische Komponenten. Vor der Ausschleusung des Wassers ist daher eine weitere chemische Behandlung erforderlich. Ein Verfahren beschreibt z.B. den umständlichen Weg der Veresterung, Hydrierung, Aminierung und Rektifikation der Ester. Die Reinigung der höheren Alkohole (beispielsweise der Oxo-Synthese) zwecks Beseitigung von Säuren, Estern, Farbstoffen und dergleichen erfolgt durch reines Natrium und Alkali-Wäsche (FR-PSS 1 493 754 = GB-PS 1 173 217 und 1 464 954 = US-PS 3 359 335).

Die Auftrennung der Alkohole in die verschiedenen gewünschten Fraktionen erfolgt durch Rektifikation.

Die Aufarbeitung nach dem Stande der Technik ist somit sehr kostenaufwendig.

Daraus ergab sich die Aufgabe, eine Aufarbeitung zu finden, bei der die kostenaufwendige destillative Abtrennung von Prozesswasser weitgehend vermieden wird. Eine weitere Aufgabe war, die nachträglichen Aufarbeitungskosten von Abwasser zu verringern bzw. zu vermeiden, damit es ohne nennenswerte chemische bzw. biologische Aufarbeitung ausgeschleust werden kann. Ferner wurde auch angestrebt, die oben erwähnte Auftrennung der höheren Alkohole unter Vermeidung von aufwendigen Kolonnen und Apparaten sinnvoll zu verwirklichen.

Diese Aufgabe wird erfindungsgemäss entsprechend den Angaben der Patentansprüche gelöst.

Gemäss der Erfindung wird aus dem homogenen Gemisch aus Methanol, Wasser und $C_{6+}$- bzw. $C_{8+}$-Alkoholen in einer ersten Destillationsstufe (K 1) nur Methanol über Kopf abgetrennt. Die Destillation erfolgt bei 500 bis 1000 mbar, vorzugsweise bei 800 bis 1000 mbar Kopfdruck. Man trennt anschliessend aus dem Sumpfablauf das Wasser, das in Abwesenheit von Methanol eine eigene Phase bildet, und zwar bei 5 bis 95°C, vorzugsweise 50 bis 80°C und Normaldruck, mechanisch ab. Überraschenderweise wurde gefunden, dass man das Wasser ohne weitere Reinigung ins Abwasser abgeben kann. Das Wasser enthält im allgemeinen nur 500 bis 1000 Gew.-ppm C, darunter etwa 50 bis 150 Gew.-ppm Methanol.

In der Alkoholphase des Sumpfablaufes verbleibt nur weniger als ein Drittel der Wassermenge des Ausgangsgemisches weiter gelöst und wird bei einer nachfolgenden Destillation (K 2) gemeinsam mit gegebenenfalls vorhandenen Paraffinen u.a. Zwischensiedern von den höheren Alkoholen befreit. Das Destillat trennt sich in einem zweiten Trennbehälter wieder in zwei Phasen. Man trennt die wässerige Phase bei einer Temperatur von 5 bis 95°C, vorzugsweise 50 bis 80°C und Normaldruck ab und führt sie gegebenenfalls in den ersten Trennbehälter zurück. Auch diese Wasserphase des Destillats der Kolonne 2 mit unter 2000 Gew.-ppm C kann ins Abwasser gegeben werden. Die Fettalkohole (Sumpfprodukt von K 2) enthalten hiernach nur noch 100 Gew.-ppm Wasser. Diese letzte Entwässerung in K 2 erfolgt bei 100 bis 500 mbar, vorzugsweise bei 100 bis 200 mbar.

In einer anschliessenden Destillationsvorrichtung (K 3 und K 4) trennt man die Fettalkohole in Fraktionen, beispielsweise in die Gemische $C_{6/10}$- bzw. $C_{8/10}$-, $C_{12/14}$- und $C_{16/18}$- bzw. $C_{16/20}$-Alkohole.

In einer ersten Kolonne (K 3) werden die $C_{6/10}$- bzw. $C_{8,10}$-Alkohole wie üblich bei 20 bis 50 mbar, vorzugsweise 35 bis 45 mbar Kopfdruck über Kopf abgetrennt. Die Trennung der $C_{12/14}$- und $C_{16/18}$- bzw. $C_{16/20}$-Fraktionen voneinander und von den Hochsiedern erfolgt erfindungsgemäss in einer einzigen Kolonne: Die $C_{12}$- und $C_{14}$-Alkohole verlassen die Kolonne über Kopf und die $C_{16/18}$- bzw. $C_{16,20}$-Alkohole werden als Seitenstrom dampfförmig dem Abtriebsteil entnommen. Die Hochsieder verbleiben im Sumpf. Die Destillation erfolgt bei 5 bis 40 mbar, vorzugsweise bei 10 bis 20 mbar.

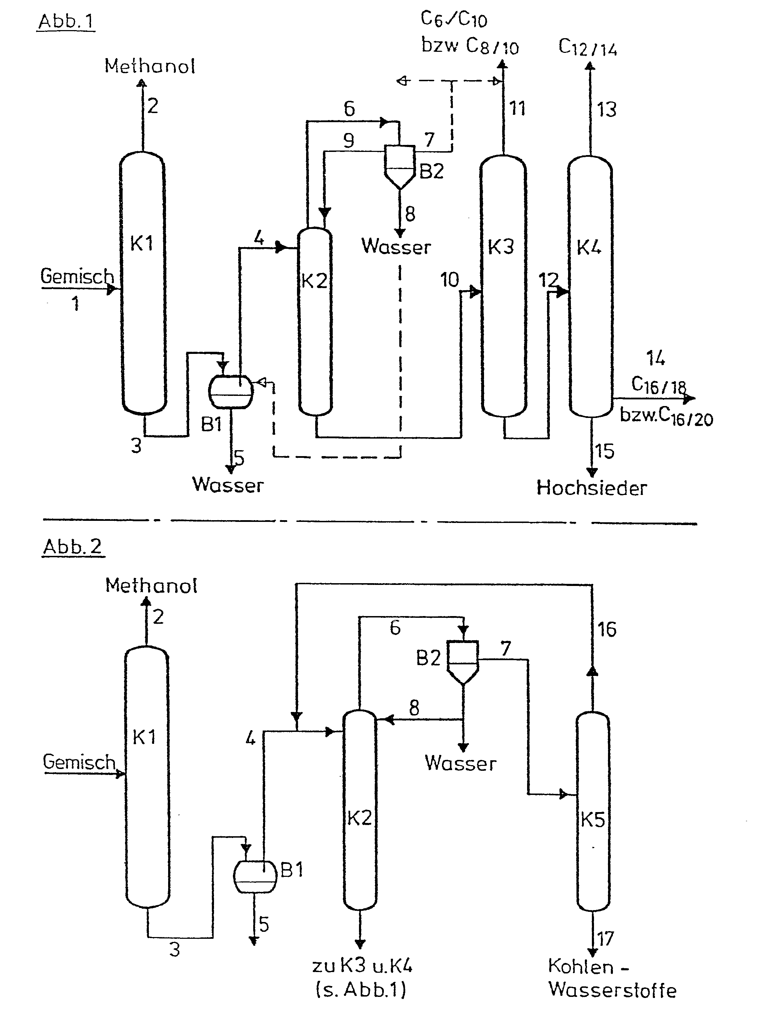

*Beispiel 1* (Abb. 1)

Ein homogenes Ausgangsgemisch der Zusammensetzung:

| | |
|---|---|
| Methanol | 32 Gew.-% |
| $C_{10+}$-Kohlenwasserstoffe | 0,2 Gew.-% |

| | |
|---|---|
| Wasser | 5 Gew.-% |
| $C_{8/18}$-Alkohole | 62,3 Gew.-% |
| und Hochsieder | 0,5 Gew.-% |

wird in die Kolonne K 1 mit 50 praktischen Böden auf den 10. Boden geführt und kontinuierlich bei 1000 mbar und einem Rücklaufverhältnis 1 destilliert. Methanol verlässt die Kolonne über Kopf (Strom 2) und enthält nur 100 Gew.-ppm Wasser. Den Sumpfablauf (Strom 3) trennt man im Trennbehälter B 1 bei Raumtemperatur in zwei Phasen. Die untere Wasserphase mit ca. 1000 Gew.-ppm C kann als Abwasser ausgeschleust werden. Die obere organische Phase mit ca. 4 Gew.-% gelöstem Wasser gelangt als Strom 4 auf den obersten Boden einer Entwässerungskolonne K 2 mit 10 praktischen Böden.

Durch Destillation bei 100 mbar wird das gelöste Wasser gemeinsam mit Oktanol und Kohlenwasserstoffen azeotrop über Kopf abgeführt und als Wasserphase im Trennbehälter B 2 bei Raumtemperatur abgeschieden. Zu Beginn werden die Destillatströme aus B 2 so lange vollständig in den Kolonnenkopf geführt, bis eine Kopftemperatur von 44°C sich einstellt. Erst danach werden die dann anfallenden Mengen als Strom 7 bzw. 8 aus B 2 entnommen. Beim stationären Zustand teilt sich der $C_8$-Alkohol zwischen dem azeotrop erforderlichen Anteil im Destillat und in Sumpf von K 2. Das Wasser, mit ca. 1500 Gew.-ppm C, kann direkt ausgeschleust werden oder in B 1 geleitet werden und als Strom 5 abgeführt werden.

Der Sumpfablauf von K 2 enthält 100 Gew.-ppm Wasser und ist methanolfrei. Er gelangt als Strom 10 auf den 10. Boden der Kolonne K 3 mit 20 praktischen Böden. Die Abtrennung der $C_{8/10}$-Alkohole über Kopf erfolgt bei einem Kopfdruck von 20 mbar und einem Rücklaufverhältnis 2. Das Destillat ist frei von $C_{10+}$-Alkoholen und der Sumpfablauf ist frei von $C_{8/10}$-Alkoholen. Er wird auf den 10. Boden der nächsten Kolonne K 4 geführt, die 25 praktische Böden hat. Die Destillation erfolgt bei 20 mbar und einem Rücklaufverhältnis von 0,33. Als Destillat erscheinen die $C_{12/14}$-Alkohole mit <1% $C_{16/18}$-Alkoholen. Die letzteren Alkohole entnimmt man der Kolonne als ein Seitenstrom am 2. Boden von unten. Der Strom 14 ist frei von $C_{12/14}$-Alkoholen und enthält <1% an Komponenten, die höher sieden als der $C_{18}$-Alkohol. Die Hochsieder verlassen die Kolonne als Sumpfablauf. Die Sumpftemperatur beträgt 240 bis 270°C, vorzugsweise 240 bis 250°C.

*Beispiel 2* (Abb. 1)

Die Auftrennung erfolgt nach den Angaben des Beispiels 1, jedoch ohne Entnahme der organischen Phase aus B 2 (Strom 7). Damit wird nur derjenige Anteil an Kohlenwasserstoffen im Kreislauf der Ströme 6 und 9 verbleiben, der aufgrund der Azeotropie erforderlich ist. Der Rest erscheint über Strom 10 im $C_{8/10}$-Schritt (Strom 11), während er im Beispiel 1 über die Wege Strom 7 sowie Strom 10 im $C_{8/10}$-Schritt erschien.

Beim Beispiel 2 findet der in der organischen Phase von B 2 gelöste Wasseranteil keinen Zugang zum Strom 11.

*Beispiel 3* (Abb. 1)

Die Auftrennung erfolgt wie im Beispiel 2. Zum Beginn der Destillation in K 2 wird aber dem Zulaufstrom 4 Hexanol zugesetzt und zwar im Verhältnis 1/340 Gewichtsanteil Hexanol je Anteil Strom 4 (Zusammensetzung von Ausgangsgemisch wie im Beispiel 1).

Die Zufuhr wird abgebrochen bei $t_{Kopf}=44°C$, wenn im Strom 9 nur wenig $C_{8+}$-Alkohole nachgewiesen werden. Das leichter siedende Hexanol übernimmt die Rolle des Schleppmittels für Kohlenwasserstoffe und Wasser und ersetzt weitgehend das Oktanol in der organischen Phase vom Trennbehälter B 2. Aufgrund der besseren Löslichkeit von Hexanol wird aber der C-Gehalt in der Wasserphase höher (ca. 3000 Gew.-ppm). Die Entnahme der Wasserphase erfolgt wie beim Beispiel 1.

Falls im Ausgangsgemisch bereits Hexanol vorkommt, so kann es z.T. nach Erreichen der Stationären Fahrweise gemeinsam mit den Kohlenwasserstoffen ausgeschieden werden.

In diesem Falle ist der Strom 11 — im Gegensatz zum Beispiel 2 — frei sowohl von Wasser als auch von Kohlenwasserstoffen.

*Beispiel 4* (Abb. 2)

Eine Ausschleusung der Kohlenwasserstoffe über Strom 7 (vgl. Abb. 1) führt zu einem gewissen Verlust an Alkoholen. Durch eine Kombination nach Abb. 2 kann der Verlust behoben werden. Die organische Phase aus B 2 wird in eine weitere Kolonne K 5 destilliert, wobei das gelöste Wasser, Alkohol und Kohlenwasserstoffe über Kopf abgehen und als Strom 16 in K 2 zugefahren werden. Bei stationärer Fahrweise enthält der Sumpfablauf nur noch Kohlenwasserstoffe (Strom 17) und ist frei von Alkoholen. Diese Fahrweise ist besonders vorteilhaft, wenn das Ausgangsgemisch einen relativ grossen Anteil an Kohlenwasserstoffen enthält. Die Auftrennung der höheren Alkohole in K 3 und K 4 erfolgt gemäss Beispiel 1.

*Beispiel 5* (Abb. 1)

Das Verfahren nach Beispiel 1 wird dahingehend vereinfacht, dass man auf die Phasentrennung im Behälter B 2 verzichtet und das gesamte Destillat von K 2 (Strom 6) in den Behälter B 2 zurückführt. Das Wasser wird nur als Strom 5 abgeschieden. Die Kohlenwasserstoffe erscheinen im Strom 11.

## Patentansprüche

1. Verfahren zur destillativen Aufarbeitung von Wasser und Methanol enthaltenden Alkoholen mit 6 bis 20 C-Atomen,
dadurch gekennzeichnet,
dass man aus der homogen wasserhaltigen Lösung zunächst durch Destillation bei einem Kopfdruck von 500 bis 1000 mbar das Methanol als Kopfprodukt abtrennt und aus dem Sumpfablauf das Wasser als eigene Phase bei Normaldruck und einer Temperatur von 5 bis 95°C in einem Trennbehälter mechanisch abtrennt, anschliessend die organische Phase vom Trenngefäss in eine zweite Destillationsvorrichtung fährt, in dieser die organische Phase bei 100 bis 500 mbar destillativ entwässert, wobei man das Kopfprodukt in einen zweiten Trennbehälter führt, die wässerige Phase bei einer Temperatur von 5 bis 95°C abtrennt und gegebenfalls in den ersten Trennbehälter zurückführt, und das Sumpfprodukt der zweiten Destillationsvorrichtung in eine anschliessende Destillationsvorrichtung fährt, in der man die $C_6$- bis $C_{20}$-Alkohole trennt.

2. Verfahren nach Anspruch 1,
dadurch gekennzeichnet,
dass man das Methanol bei einem Kopfdruck von 800 bis 1000 mbar abtrennt.

3. Verfahren nach Anspruch 1 und 2,
dadurch gekennzeichnet,
dass man die destillative Entwässerung in der zweiten Destillationsvorrichtung bei 100 bis 200 mbar durchführt.

4. Verfahren nach den Ansprüchen 1 bis 3,
dadurch gekennzeichnet,
dass man das Wasser bei einer Temperatur von 50 bis 80°C abtrennt.

5. Verfahren nach den Ansprüchen 1 bis 4,
dadurch gekennzeichnet,
dass man bei der destillativen Trennung der $C_6$- bis $C_{20}$-Alkohole zunächst die $C_6$- bis $C_{10}$-Alkohole bei 20 bis 50 mbar, vorzugsweise 35 bis 455 mbar Kopfdruck destillativ über Kopf abtrennt, anschliessend bei 5 bis 40 mbar, vorzugsweise 10 bis 20 mbar Kopfdruck die $C_{12/14}$-Alkohole als Kopfprodukt und die $C_{16,20}$-Alkohole als Seitenstrom in einer weiteren Kolonne gewinnt und die Hochsieder als Sumpfablauf ausschleust.

6. Verfahren nach den Ansprüchen 1 bis 5,
dadurch gekennzeichnet,
dass man die organische Phase aus dem zweiten Trennbehälter in einer zusätzlichen Destillationsvorrichtung (K 5) destilliert und die Kohlenwasserstoffe ausschleust.

## Claims

1. A process for working up by distillation higher alcohols of 6 to 20 carbon atoms containing water and methanol, characterised in that the mathanol is first separated as overhead product from the homogenous water-containing solution by distillation at an overhead pressure of 500 to 1000 mbar and the water is separated mechanically as an individual phase from the bottoms residue at standard pressure and a temperature of 5 to 95°C in a separation vessel, subsequently the organic phase is passed from the separation vessel to a second distillation apparatus in which the organic phase is dewatered by distillation at 100 to 500 mbar, the overhead product being passed to a second separation vessel where the aqueous phase is separated at a temperature of 5 to 95°C and optionally returned to the first separation vessel, and the bottoms product of the second distillation apparatus is passed to a subsequent distillation apparatus in which the $C_6$ to $C_{20}$ alcohols are separated.

2. A process according to claim 1, characterised in that the methanol is separated at an overhead pressure of 800 to 1000 mbar.

3. A process according to claim 1 or 2, characterised in that the dewatering by distillation is carried out in the second distillation apparatus at 100 to 200 mbar.

4. A process according to any of claims 1 to 3, characterised in that the water is separated at a temperature of 50 to 80°C.

5. A process according to any of claims 1 to 4, characterised in that in the separation of the $C_6$ to $C_{20}$ alcohols by distillation first the $C_6$ to $C_{10}$ alcohols are separated as overhead product by distillation at an overhead pressure of 20 to 50 mbar, preferably 35 to 45 mbar, and subsequently in a further colomn at an overhead pressure of 5 to 40 mbar, preferably 10 to 20 mbar, the $C_{12}$ to $C_{14}$ alcohols are obtained as overhead product and the $C_{16}$ to $C_{20}$ alcohols as a side stream, the high-boiling material being discarded as bottoms residue.

6. A process according to any of claims 1 to 5, characterised in that the organic phase from the second separation vessel is distilled in an additional distillation apparatus (K5) and the hydrocarbons are discarded.

## Revendications

1. Procédé pour le traitement par distillation d'alcools supérieurs ayant de 6 à 20 atomes de carbone et contenant de l'eau et du méthanol, caractérisé par le fait que de la solution homogène contenant de l'eau, on sépare tout d'abord par distillation le méthanol comme produit de tête, à une pression de tête de 500 à 1000 mbars et que, de l'écoulement de queue, on sépare mécaniquement l'eau comme phase particulière, à la pression normale et à une température de 5 à 95°C dans un séparateur, qu'ensuite on amène la phase organique du séparateur à un deuxième appareil de distillation, que dans celui-ci on déshydrate par distillation la phase organique à une pression de 100 à 500 mbars en amenant le produit de tête à un deuxième séparateur, que l'on sépare la phase aqueuse à une température de 5 à 95°C et qu'éventuellement on la ramène au premier séparateur et que l'on amène le produit de queue du deuxième appareil de distillation à un appareil de distillation qui fait suite, dans lequel on sépare les alcools en $C_6$ à $C_{20}$.

2. Procédé selon la revendication 1, caractérisé par le fait que l'on sépare le méthanol à une pression de tête de 800 à 1000 mbars.

3. Procédé selon les revendications 1 et 2, caractérisé par le fait que l'on conduit la déshydratation par distillation dans le deuxième appareil de distillation à une pression de 100 à 200 mbars.

4. Procédé selon les revendications 1 à 3, caractérisé par le fait que l'on sépare l'eau à une température de 50 à 80°C.

5. Procédé selon les revendications 1 à 4, caractérisé par le fait que lors de la séparation par distillation des alcools en $C_6$ à $C_{20}$, on sépare tout d'abord par distillation à la tête les alcools en $C_6$ à $C_{10}$, à une pression de tête de 20 à 50 mbars, de préférence de 35 à 45 mbars, qu'ensuite, à une pression de tête de 5 à 40 mbars, de préférence de 10 à 20 mbars, on obtient dans une autre colonne les alcools en $C_{12.14}$ comme produit de tête et les alcools en $C_{16/20}$ comme produit de tête et les alcools en $C_{16/20}$ comme courant latéral et qu'on élimine les corps à point d'ébullition élevé comme écoulement de queue.

6. Procédé selon les revendications 1 à 5, caractérisé par le fait que dans un appareil de distillation supplémentaire (K 5), on distille la phase organique venant du deuxième séparateur et que l'on élimine les hydrocarbures.

Abb. 1

Abb. 2